# EUROPEAN PATENT APPLICATION

(11) **EP 4 613 866 A1**
(43) Date of publication of application: **10.09.2025**
(21) Application number: 23883965.8
(22) Date of filing: 06.11.2023
(51) Int. Cl.: C12N 15/87, C12N 5/00, A61K 35/51

(54) **COMPOSITION OF MITOCHONDRIA ENRICHED WITH MITOCHONDRIAL ANTIVIRAL-SIGNALLING PROTEIN (MAVS) FOR TREATING VIRAL DISEASES**

(30) Priority: 04.11.2022 CL 20223057
(71) Applicant: Cells For Cells S.A., Santiago 7550101 (CL)
(72) Inventor: CUENCA MORENO, Jimena, Santiago, 7550101 (CL); CASTILLO GALÁN, Sebastián, Santiago, 7550101 (CL); KHOURY, Maroun, Santiago, 7550101 (CL)
(74) Representative: Elzaburu S.L.P.
(86) International application number: PCT/CL2023/050103
(87) International publication number: WO 2024/092375

(57) **Abstract**

The invention relates to a method for obtaining a composition of mitochondria isolated from umbilical cord blood mesenchymal/stromal cells (UC-MSC) enriched in the mitochondrial antiviral signaling protein (MAVS), and the use of this composition of mitochondria enriched in MAVS for the treatment of viral diseases.

Where the method for obtaining a composition enriched in mitochondria with active MAVS from mesenchymal cells comprises providing a culture of mesenchymal cells seeded at a density between 500 and 15,000 cells/cm²; inducing enrichment in mitochondrial antiviral signaling protein (MAVS) in the mitochondria of said cells and incubating for 1 to 5 hrs; c) extracting the mitochondria enriched in mitochondrial antiviral signaling protein (MAVS); d) suspending the mitochondria with active MAVS in a vehicle, clinical grade buffer, obtaining the composition enriched in mitochondria. The use of the obtained composition to counteract the effects of viral infections is also protected.

## Description

The invention relates to a method for obtaining a composition of mitochondria isolated from umbilical cord mesenchymal/stromal cells (UC-MSC) which have been enriched in the mitochondrial antiviral signaling protein (MAVS), and to the use of this composition of mitochondria enriched in MAVS for the treatment of viral diseases.

### Previous background

Mitochondria are now well recognized as key components in controlling signaling pathways essential for combating viral infections. Although several pathogen-activated pathways are influenced by mitochondria, this role can primarily be attributed to the mitochondrial localization of the mitochondrial antiviral signaling adaptor protein (MAVS, also known as IPS1, VISA, and CARDIF), a key mediator of the innate immune response during RNA virus infection. Upon viral recognition, the mitochondrial antiviral signaling protein induces the activation of interferon and nuclear factor-κB (NF-κB) transcription factors. This process ultimately leads to the expression of multiple proinflammatory factors and antiviral genes, such as IFNy and IFN-stimulated genes (ISGs), which inhibit viral replication and transmission, playing an important role in antiviral defense.

It has recently been demonstrated that mitochondria can be transferred from one cell type to another through various mechanisms, with metabolic and functional consequences for the cells that receive the mitochondria, reinforcing the current interest in the biological properties of these organelles.

Additionally, it has been shown that mitochondria isolated from cells can be transplanted into other cells or tissues, incorporated, and remain functional in the recipient cells. For example, the MitoCeption protocol allows the transfer of mitochondria previously isolated from donor cells to target cells. Thus, mitoception, or other methods of incorporating functional mitochondria into different target cells, have begun to be used in various therapies, mainly in cases where damage, reduction, or lack of mitochondria has been observed in the target cells or tissues.

For example, the publication WO2023033761A1 (OVALI ERCUEMENT [TR]; BAGIS HALIL TAYFUN [TR] 2023-03-09), discloses a method for increasing fertility through the "transplantation" of isolated mitochondria mediated by macropinocytosis, via incubation with germinal vesicle (GV) oocytes in meiosis phase 1 and oocytes in meiosis phase 2. This is based on the knowledge that the number of mitochondria per cell is quite important for meiosis division. Unless the number of mitochondria in an oocyte reaches a certain level, it is believed that these oocytes cannot reach meiosis.

A second example is the publication WO2020202103A1 (MOR RESEARCH APPLIC LTD [IL], 2020-10-08), which describes methods for the treatment of ocular diseases and conditions associated with mitochondrial dysfunction, mitochondrial destruction, and/or mitochondrial depletion in the eye. These methods involve the transplantation of isolated, exogenous, and viable mitochondria into ocular tissues. Mitochondria are administered directly to the eye by injection into the vitreous, cornea, ocular muscle, anterior chamber, or suprachoroidal space; or by topical application to the ocular surface.

Thus, the disclosed treatments that deliver mitochondria thus far seek to compensate for a mitochondrial deficiency in the target cell or tissue being treated.

In this context, the inventors' proposal is disruptive, as it does not seek to increase the number of mitochondria in a recipient cell, but rather to activate, reactivate, or enhance the recipient cells' own antiviral mechanisms by incorporating healthy mitochondria enriched with the mitochondrial antiviral signaling adaptor protein, triggering, enhancing, or accelerating the cell's antiviral response.

The inventors have developed a method for obtaining a composition containing mitochondria enriched with MAVS, which can be used as therapy for acute viral infections. The composition is administered parenterally. This composition may be useful in the treatment of respiratory viral diseases, such as those caused by RNA viruses: syncytial virus, influenza virus, parainfluenza virus, metapneumovirus, rhinovirus, and coronavirus, among others.

### Description of the figures.

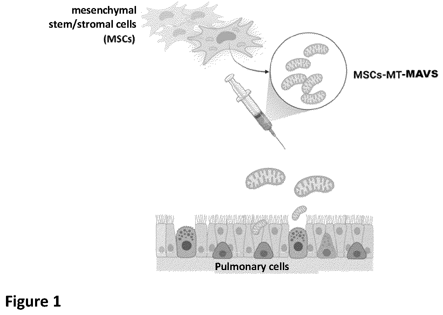

**Figure 1****. Experimental scheme.** Mitochondria (MT) enriched in MAVS are isolated from UC-MSC, obtaining the composition of the invention. The mitochondria of the composition of the invention are naturally internalized by mitoception in target cells, for example, pulmonary cells with an activated viral state
**Figure 2. Determination of the functional state of isolated mitochondria and internalization.** A) Mitochondrial membrane potential measurement using TMRE (tetramethylrhodamine ethyl ester; at two different concentrations: 0.4 and 1 µM) in isolated mitochondria (MT) derived from umbilical cord blood mesenchymal stem/stromal cells (UC-MSCs). CCCP (carbonyl cyanide m-chlorophenyl hydrazone) is used as a control, as it induces mitochondrial dysfunction. B) Representative graph showing flow cytometry of the internalization of MAVS-enriched MT (MT^{MAVS}) isolated from UC-MSCs. The MT^{MAVS} were previously stained with Mitotracker green, then incorporated into virally activated A549 lung cells (250 ng/ml vRNA) and analyzed by flow cytometry. C) The percentage of positive cells that internalized these MAVS-enriched MT is shown (left panel) and the mean fluorescence intensity (MFI) was also determined (right panel)
**Figure 3. Characterization of isolated mitochondria derived from UC-MSC.** A fraction of mitochondria enriched in MAVS was obtained from UC-MSCs. A) Representative images of transmission electron microscopy (TEM) of this fraction are shown. B) Adenosine triphosphate (ATP) detection using a commercial kit. C) Determination of mitochondrial membrane potential using tetramethylrhodamine ethyl ester (TMRE) staining by flow cytometry. E) Sodium dodecyl sulfate agarose gel electrophoresis (SDS-AGE) showing MAVS aggregation in isolated mitochondria. F) MAVS protein expression determined by western blot. Bars represent the mean +/- SEM, 1-3 different UC-MSC donors
**Figure 4. Activation of the MAVS antiviral pathway in UC-MSC cells.** Cells were stimulated with a MAVS activator. Western blot of cell lysate to detect A) MAVS1 B) MAVS 2 and C) the MAVS1/MAVS2 ratio; D) Representative images of MAVS Western Blot. E) Densitometry of IRF3, F) p-IRF3 and G) the p-IRF3/IRF3 ratio. H) Representative images of the western blot. I-L) Relative gene expression of genes: MAVS, and those related to interferon (INFL2, IL8; Serpin1), determined by qRT-PCR. LL-P) Western blot analysis of proteins involved in fusion (Mfn1, OPA1), fission (DRP1) and mitophagy (Parkin). Q) Representative images of Western Blot. The bar represents the mean +/- SEM; 3 different UC-MSC donors. (n=3 independent experiments). *p≤0.05, **p≤0.01. U.A. Arbitrary units
**Figure 5. Effects of MAVS activator stimulation on UC-MSC.** A) ATP production in UC-MSC was determined by a commercial kit, the graph shows the ATP produced, oligomycin (OLN) inhibition is used as a control. B) Cellular reactive oxygen species (ROS) in UC-MSC measured as MFI of dihydrodichlorofluorescein diacetate (DCF). C) Representative histograms of ROS in UC-MSC cells. D) Flow cytometry analysis of apoptosis/necrosis. E) Representative dot plot of flow cytometry of apoptosis/necrosis. 3 different UC-MSC donors. Bars indicate the mean +/- SEM (n=3 independent experiments). *p≤0.05. A.U.: Arbitrary units.
**Figure 6. Determination of internalization of MT^{MAVS} in pulmonary cells activated to a viral state.** MAVS-enriched MT (MT^{MAVS}) isolated from MSCs were stained with MitoTracker Green and then incorporated into virally activated A549 pulmonary cells at different RNA concentrations (250 and 500 ng/mL). A) The percentage of positive cells that internalized these MAVS-enriched MT, and B) the mean fluorescence intensity (MFI) is shown. C) Representative graph showing flow cytometry of internalization.
**Figure 7. The incorporation of MAVS-enriched mitochondria increases the viability and decreases oxidative stress of activated pulmonary cells to a viral state.** A549 pulmonary cells were activated with different concentrations of artificial viral RNA (250 and 500 ng/ml), and then treated with the composition of the invention, with mitochondria enriched in MAVS (MT^{MAVS}). A) Percentage of cell viability and B) Determination of reactive oxygen and nitrogen species by DCF. The bars indicate the mean +/- SEM (n = 3 independent experiments). **p≤0.01, ***p≤0.001 y ****p≤0.0001.
**Figure 8. The incorporation of mitochondria enriched in MAVS increases the viability of activated pulmonary cells to a viral state.** A549 pulmonary cells were activated with different concentrations of artificial viral RNA (250 and 500 ng/ml) and then treated with the composition of the invention, containing mitochondria enriched in MAVS (MT^{MAVS}) in a 4:1 ratio for 24 hours. A) % necrotic cells; B) % cells in late apoptosis; C) % cells in early apoptosis; and D) % live cells determined by 7ADD/Annexin V by flow cytometry. Bars represent the mean +/- SEM. **p≤0.01, ***p≤0.001 y ****p≤0.0001. One-way ANOVA, Newman-Keuls post-test.
**Figure 9. Incorporation of MAVS-enriched mitochondria restores the membrane potential and ATP production of Pulmonary cells activated to a viral state.** A549 pulmonary cells were activated with different concentrations of artificial viral RNA (250 and 500 ng/ml) and then treated with the composition of the invention, with mitochondria enriched in MAVS (MT^{MAVS}). A) MT membrane potential with TMRE (tetramethylrhodamine, ethyl ester, active mitochondria label) and B) ATP production measured with the commercial CellTiter-Glo Luminescent Cell Viability Assay kit. The bars represent the mean +/- SEM. **p≤0.01, ***p≤0.001 y ****p≤0.0001. One-way ANOVA, Newman-Keuls post-test.
**Figure 10. Distribution of MSC-MT^{MAVS} after intravenous administration in healthy mice.** MSC were stained with Mitoview 720 and subsequently the mitochondria enriched in MAVS were isolated. The composition of the invention of mitochondria enriched in MAVS was injected through the tail vein very slowly and the different organs and blood were extracted and analyzed at different times (n = 3 animals per group). A) Fluorescence analysis in different organs at 10 min, 2 h, 4 h and 24 h using the Oddysey ^{®} CLx system. B) Representative images of the organs of mice injected with labeled MSC-MT^{MAVS}. C) Analysis strategy of the leukocyte population in the blood of mice. The blood of the mice was stained with α-CD45, α-CD3, α-CD19, α-CD14. Sample analysis was performed by flow cytometry on a FACSCanto^{™} II cytometer (BD Biosciences). D) Detection of mitochondria in mouse blood leukocytes after intravenous injection. The geometric mean fluorescence of MitoView^{™} 720 (Biotium) (relative to control) in the blood leukocyte population was measured by flow cytometry after 10 min, 2 h, 4 h, and 24 h of intravenous injection of buffer (CTRL), or MSC-MT^{MAVS}. Bars represent the mean +/- SEM.

### Description of the invention.

As indicated, the invention relates to a method for obtaining a composition of mitochondria isolated from umbilical cord blood mesenchymal stem/stromal cells (UC-MSC) enriched in mitochondrial antiviral signaling protein (MAVS), to the composition with MAVS-enriched mitochondria, and to the use of this composition of MAVS-enriched mitochondria for the treatment of viral diseases.

To obtain the composition of the invention, the inventors work with healthy cells, specifically with mesenchymal stem/stromal cell (MSC) cultures, which in a preferred embodiment are umbilical cord blood mesenchymal stem/stromal cells (UC-MSC). These MSC cultures are treated or manipulated with any method known in the art to induce an increase in MAVS expression, for example, synthetic commercial molecules such as RNA, lentiviruses, plasmids, or others.

The inventors have established that treatment with the composition of the invention improves cell viability in virus-infected tissues, improving various parameters associated with viral infection. We have established these improvements for a human cell line, the A549 pulmonary cell line, not with real viruses but with an RNA generated by in vitro transcription of a sequence from the H1N1 virus. This RNA activates various signaling pathways that are also activated by viruses and induce cellular antiviral activity.

Mitochondria enriched in MAVS are isolated from the cells and suspended in a vehicle, a clinical-grade carrier, such as a buffer, and constitute the composition of the invention, which can be used as a therapy to help combat viral infections.

It will be evident to those skilled in the art that the specific nature of the buffer used is irrelevant as long as it is suitable for clinical-grade injection into mammals and allows mitochondrial viability. Commercial buffers are available for this purpose and can be used interchangeably in the composition of the invention, given that the active component is MAVS-enriched mitochondria.

Thus, the invention relates to a method for obtaining a mitochondria-enriched composition with active MAVS from mesenchymal cells, comprising:
a) providing a culture of mesenchymal cells seeded at a density between 500 and 15,000 cells/cm²
b) inducing mitochondrial antiviral signaling protein (MAVS) enrichment in the mitochondria of said cells and incubating for 1 to 5 hours;
c) extracting mitochondria enriched with mitochondrial antiviral signaling protein (MAVS);
d) suspending the mitochondria with active MAVS in a vehicle, clinical-grade buffer, obtaining the composition enriched in mitochondria with active MAVS. Where the clinical-grade buffer vehicle is preferably a mitochondrial respiration medium.

Where in step b, MAVS enrichment is induced by adding lentiviruses, plasmids, isolated viral RNA molecules, or synthetic RNA to the culture medium. In a preferred embodiment, 3p-hpRNA is used at a concentration of 100 to 400 ng/mL in the culture medium.

Preferably, the mesenchymal cells used are umbilical cord mesenchymal cells.

In a second aspect, the invention relates to a mitochondria-enriched composition from mesenchymal cells comprising isolated mitochondria with increased expression of mitochondrial antiviral signaling protein (MAVS) in a carrier vehicle, a clinical-grade buffer. The clinical-grade buffer vehicle is preferably a mitochondrial respiration medium.

In a third aspect, the invention relates to the use of said composition of the invention to prepare a medicine useful for counteracting the effects of viral infections. The medicine is for parenteral or systemic administration.

This medication activates the antiviral response in virus-infected cells and counteracts the effects of acute viral infections. It particularly counteracts the effects of respiratory viral infections. And very specifically, it counteracts the effects of respiratory viral infections caused by RNA viruses such as syncytial virus, influenza virus, parainfluenza virus, metapneumovirus, rhinovirus, or coronavirus.

The invention will be better understood from the following illustrative examples.

### Examples

### Example 1. Obtaining the composition of the invention.

As indicated, the inventors conducted studies with a composition of mitochondria enriched in MAVS obtained from UC-MSC for the treatment of viral diseases. These studies used an in vitro model in which lung cells treated with an artificial RNA of viral origin were treated with the composition of the invention, a composition of mitochondria enriched in MAVS derived from UC-MSC.

To provide greater clarity regarding the assays, a representative image of the experimental design can be seen in Figure 1.

In a 150mm dish, umbilical cord blood mesenchymal stromal cells were cultured at an initial density of 2,000 cells/cm² (3-6 passages). This was done in 12 mL of low-glucose DMEM medium supplemented with 10% FBS, 1X L-glutamine, and the antibiotic penicillin/streptomycin 1X. When the cells reached 70-80% confluence, they were transfected with 3p-hpRNA at a concentration of 250 ng/mL.

3p-hpRNA is a 5' triphosphate hairpin RNA generated by in vitro transcription of an influenza A (H1N1) virus sequence and can be used as an inducer of antiviral responses, including the expression of MAVS in cell mitochondria.

The cells were subsequently incubated in an incubator at 37°C and 5% CO2 for 2 hours to induce aggregation and activation of MAVS.

Mitochondria were then isolated using the Mitochondria Isolation Kit for Mammalian Cells (Thermo Scientific, IL, USA), following the manufacturer's instructions. Briefly, the cultures were dissociated to remove the cells from the plate, washed, and centrifuged (2 minutes at 1500 g). The pellets, containing 1x10⁶ of UC-MSC, were homogenized with 200 µL of the kit's reagent A and 2 µL of the protease inhibitor (Halt Protease and Phosphatase Inhibitor Cocktail, EDTA-Free, Thermo Scientific), vortexed for 5 seconds. The pellets were then incubated on ice for 2 minutes. Subsequently, 2.5 µL of reagent B from the Kit was added and homogenized by vortexing for 10 seconds every 30 seconds, for 5 minutes, incubating on ice in between. Then, 200 µL of reagent C from the Kit and 2 µL of the protease inhibitor were added, the contents were mixed by inverting the tube slightly for 30 seconds. The tube was centrifuged at 700g for 10 minutes and the supernatant was transferred to a new sterile 1.5 mL Eppendorff tube. This new tube was centrifuged at 3,000 g for 15 minutes. Finally, the supernatant (cytosolic fraction) was discarded and the pellet (mitochondrial fraction) was resuspended in 0.2-1 mL of a clinical grade buffer composition (vehicle), specifically a Mitochondrial Respiration Medium.

Where said mitochondrial respiration medium, or vehicle, has the following composition:

**Table 1**

| | Final Concentration [Mm] |
|---|---|
| EGTA | 0.5 |
| MgCl₂ | 3 |
| Lactobionic acid | 60 |
| Taurine | 20 |
| KH₂PO₄ | 10 |
| HEPES | 20 |
| D-Sucrose | 110 |
| BSA, fatty acid free | 1 g/L |

Where isolated mitochondria enriched with MAVS, suspended in a suitable clinical-grade buffer solution, as shown in Table 1, constitute the composition of the invention.

To determine the functional state of the mitochondria isolated in the composition of the invention, membrane potential was measured (Figure 2 A). TMRE (tetramethylrhodamine ethyl ester; at two different concentrations: 0.4 and 1 µM) was used in the composition of the invention (MT), in the presence (100) and absence of CCCP (carbonyl cyanide m-chlorophenyl hydrazone) as a control, as it induces mitochondrial dysfunction.

TMRE emits a red fluorescence that can be detected by flow cytometry or fluorescence microscopy, and the level of TMRE fluorescence in mitochondria correlates with mitochondrial functionality. In this case, it can be seen that mitochondria significantly decrease their functionality in the presence of CCCP, demonstrating that the MT in the composition of the invention are functional.

To assess whether the mitochondria of the invention are internalized into recipient cells, the MTMAVS were stained with Mitotracker green, then incorporated into A549 pulmonary cells activated to a viral state (250 ng/ml vRNA) and analyzed by flow cytometry. The results are shown in Figure 2 B and C. Figure 2 B shows the flow cytometry of untreated cells and cells treated with MT MAVS, which is graphed in Figure 2 C, with the percentage of cells positive to the fluorescent marker or expressed as fluorescence intensity (MFI).

This demonstrates that the composition of the invention is efficiently incorporated into target cells by mitoception.

To assess whether mitochondria are enriched in MAVS, mitochondrial fractions were isolated from UC-MSC exposed to 250 ng/mL of 3p-hpRNA. It was determined that the isolated mitochondria derived from UC-MSCs and enriched in MAVS displayed MAVS protein expression **(Figure 3F)** and MAVS aggregates, indicating that MAVS is activated, which is not observed in control mitochondria **(Figure 3E).** These results directly and indirectly indicate that the addition of 3p-hpRNA induces MAVS aggregation in mitochondria.

Transmission electron microscopy (TEM) confirmed that in mitochondrial isolates from UC-MSC, mitochondria retain their morphology and size **(Figure 3A-B).** Their functionality was assessed through ATP production as well as mitochondrial membrane potential **(Figure 3C-D).** It was also determined that both remained without significant changes between MAVS-enriched mitochondria and the control. As previously indicated, SDS-AGE determined that mitochondria derived from UC-MSC and enriched in MAVS, once isolated, presented activated MAVS or MAVS aggregates, which are not observed in controls **(Figure 3E).**

Additionally, it was demonstrated that the parental cells, the UC-MSCs, when stimulated with the MAVS activator, increased MAVS gene and protein expression and that they activated the pIRF3/IRF3 pathway, also inducing interferon (IFN) and the expression of IFN-inducible genes (ISG) such as interleukin 8 (IL8) and serpin 1. (SERPIN1) (Figure 4A-L). These results indicate that exposure to 3p-hpRNA induces the activation of genes and proteins that are associated with MAVS activation, confirming their activation.

Studies related to changes in mitochondrial dynamics resulting from the enrichment of MAVS in the mitochondria of UC-MSC suggest that these remain without relevant changes. When the expression of proteins involved in fusion was evaluated: Mitofusin 1 (Mfn1), Optic atrophy-1 (OPA1), fission: Dynamin-related protein 1, isoform A (DRP1) and mitophagy: Parkin, these did not present changes with respect to the control **(Figure 4** LL-Q). Regarding functional aspects of cells related to mitochondrial activity, we observed that the production of ATP and ROS in stimulated UC-MSC did not present changes in relation to the control **(Figure 5A-C).** Finally, it was determined that the stimulus does not have an impact on cell viability as observed in Figure 5D-E, where the percentages of apoptosis, necrosis and viability remain similar with respect to the control. Taken together, these data indicate that the stimulus used in UC-MSC promotes enrichment in MAVS and that they are capable of activating the IRF pathway and the expression of ISG-associated genes.

### Example 2. In vitro effectiveness study

The inventors conducted various *in vitro* studies with pulmonary cells activated to a virally infected state.

To this end, a pulmonary cell culture was treated with an artificial RNA of viral origin (vRNA). Two concentrations of viral vRNA were tested: 250 and 500 ng/mL. The composition of the invention was added to the control (without vRNA) and to cultures treated with each concentration of vRNA.

Various functional analyses were performed on pulmonary cells treated with the composition of the invention, containing mitochondria enriched in MAVS (MT^{MAVS}). It was determined that more than 70% of pulmonary cells, whether control or activated to the viral state, internalized MT^{MAVS} (Figure 6). Cell viability, reactive oxygen and nitrogen species (ROS), ATP, and membrane potential, all parameters that undergo changes due to viral infection, were also determined.

In the results presented in Figure 7A, it can be observed that treatment of mitochondria enriched in MAVS with the composition of the invention increased the viability of pulmonary cells treated with vRNA, reaching levels close to control levels at both concentrations of vRNA tested. It should be noted that treatment with vRNA decreased cell viability, which was reversed in both cases with the composition of the invention. Figure 7B shows that in the condition with vRNA, oxidative stress increases, measured with the DCF test (Dihydrodichlorofluorescein diacetate), said stress is also significantly reduced with the composition of the invention, in the 2 conditions studied.

These results demonstrate that the composition of the invention is useful in reversing the viral pathological state in pulmonary cells.

The results presented in Figure 8 show that treatment of MAVS-enriched mitochondria with the composition of the invention produces a decrease in the percentage of cells in a state of necrosis, late apoptosis, and early apoptosis in pulmonary cells treated with vRNA, returning to levels close to control levels at both concentrations of RNA tested (Figure 8 A-D). It should be noted that treatment with viral RNA alters all the parameters analyzed in this assay, which is reversed with the composition of the invention.

Figure 9 shows parameters such as mitochondrial membrane potential, measured with the tetramethylrhodamine ethyl ester (TMRE) probe, and adenosine triphosphate (ATP) levels. Exposure to vRNA, at both concentrations, reduces mitochondrial membrane potential and decreases ATP production (Figure 9, A-B). However, these parameters are reversed with the composition of the invention, under both RNA conditions studied, increasing membrane potential and ATP levels.

All these results demonstrate that treatment with the composition of the invention can reverse the effects of a viral infection.

### Example 3. In vivo distribution study of the composition of the invention

To analyze the *in vivo* distribution of the composition of the invention of mitochondria enriched in MAVS, healthy C57BL/6 mice were used, to which were administered systemically via the tail vein, 200 mL of:
I) Composition of the Invention: Mitochondria enriched in MAVS, from 1x10⁶ UC-MSC, pre-stained with MitoView720, in clinical grade buffer solution, as a vehicle for mitochondria.
II) Control: Clinical grade buffer solution + supernatant obtained from mitochondrial isolation (1:1).

The results show that systemic administration of the inventive composition of mitochondria enriched in MAVS did not induce adverse reactions related to mortality. No traces of ischemia or infarction were found in the necropsies of each experimental group. The animals exhibited behavior similar to control animals.

The MitoView 720 stain, which represents the administered stained mitochondria, was observed in the brain, heart, lung, liver, spleen, kidneys, intestines, axillary nodes, and blood samples. Overall, we highlight the persistence and increase of the stain in the heart, lung, and spleen 24 hours after administration. This suggests that the inventive composition of mitochondria enriched in SAM, after 24 hours, remains in organs relevant to the study of viral infections, such as the lungs (Figure 10). Blood studies do not show any labeling in the different circulating cell populations such as CD45 (leukocytes), CD3 (T lymphocytes), CD19 (B lymphocytes), and CD14 (monocytes), showing that these cells would not capture these mitochondria, and that these are adopted in the organs (Figure 10).

These results confirm that treatment with the composition of the invention, containing mitochondria enriched in MAVS, reduces the damage generated in pulmonary cells activated by viral RNA.

The *in vitro* results demonstrate a significant improvement in viral pathology resulting from treatment with the composition of the invention, containing mitochondria enriched in MAVS. In *vivo* data in a mouse model indicates that the composition of the invention, administered intravenously (systemically), can remain in the animal's organs for up to 24 hours, showing no adverse effects.

Based on the above, it can be concluded that the production methodology of a composition of mitochondria enriched with MAVS obtained from UC-MSC generates a beneficial effect on acute viral lung infections, such as pneumonia, or any other acute and severe viral infection, such as those caused by RNA viruses: syncytial virus, influenza virus, parainfluenza virus, metapneumovirus, rhinovirus, and coronavirus, among others.

The invention therefore relates to a method of treating a viral infection, which comprises applying the composition of the invention parenterally or systemically in order to access the tissues affected by the viral infection.

In one embodiment, the mesenchymal cells used for this methodology correspond to umbilical cord mesenchymal cells.

## Claims

1. Method for obtaining a mitochondria-enriched composition with active MAVS from mesenchymal cells, **CHARACTERIZED in that** it comprises:
a) providing a culture of mesenchymal cells seeded at a density between 500 and 15,000 cells/cm²
b) inducing enrichment of mitochondrial antiviral signaling protein (MAVS) in the mitochondria of said cells and incubating for 1 to 5 hrs;
c) extracting the mitochondria enriched with mitochondrial antiviral signaling protein (MAVS);
d) suspending the mitochondria with active MAVS in a vehicle, clinical-grade buffer, obtaining the mitochondria-enriched composition.

2. Method according to claim 1 **CHARACTERIZED in that** in step b the antiviral response is induced by adding lentiviruses, plasmids, isolated viral RNA molecules, or synthetic RNA to the culture medium.

3. Method according to claim 2, **CHARACTERIZED in that** 3p-hpRNA is the RNA used.

4. Method according to claim 3, **CHARACTERIZED in that** 3p-hpRNA is used at a concentration between 100 and 400 ng/mL in the culture medium.

5. Method according to claim 1, **CHARACTERIZED in that** the mesenchymal cells used are umbilical cord mesenchymal cells.

6. Method according to claim 1, **CHARACTERIZED in that** the clinical-grade buffer vehicle is a mitochondrial respiration medium.

7. Composition enriched with activated mitochondria from mesenchymal cells, **CHARACTERIZED in that** it comprises isolated mitochondria with increased expression of mitochondrial antiviral signaling protein (MAVS) in a carrier vehicle, clinical-grade buffer.

8. Composition according to claim 7, **CHARACTERIZED in that** the clinical-grade buffer vehicle is a mitochondrial respiration medium.

9. Use of the composition of claim 7, **CHARACTERIZED in that** it allows the preparation of a medicine useful for counteracting the effects of viral infections.

10. Use according to claim 9, **CHARACTERIZED in that** the medicine is for parenteral or systemic administration.

11. Use according to claim 9, **CHARACTERIZED in that** the medicament activates the antiviral response in virus-infected cells.

12. Use according to claim 9, **CHARACTERIZED in that** it counteracts the effects of acute viral infections.

13. Use according to claim 12, **CHARACTERIZED in that** it counteracts the effects of respiratory viral infections.

14. Use according to claim 13, **CHARACTERIZED in that** it counteracts the effects of respiratory viral infections caused by RNA viruses such as syncytial virus, influenza virus, parainfluenza virus, metapneumovirus, rhinovirus, or coronavirus.
